# EUROPÄISCHE PATENTSCHRIFT

(11) **EP 3 506 812 B1**
(45) Veröffentlichungstag und Bekanntmachung des Hinweises auf die Patenterteilung: **29.09.2021**
(21) Anmeldenummer: 17758458.8
(22) Anmeldetag: 15.08.2017
(51) Int. Cl.: A61B 1/12, A61B 1/00, A61L 2/24

(54) **AUFBEREITUNG VON CHIRURGISCHEN INSTRUMENTEN, INSBESONDERE ENDOSKOPEN**
PREPARING SURGICAL INSTRUMENTS, IN PARTICULAR ENDOSCOPES
PRÉPARATION D'INSTRUMENTS CHIRURGICAUX, NOTAMMENT D'ENDOSCOPES

(30) Priorität: 31.08.2016 DE 102016216403
(43) Veröffentlichungstag der Anmeldung: 10.07.2019
(73) Patentinhaber: Olympus Winter & Ibe GmbH, 22045 Hamburg (DE)
(72) Erfinder: THATE, Henning, 22417 Hamburg (DE); LENGSFELD, Michael, 22179 Hamburg (DE); FRICK, Eugen, 21031 Hamburg (DE)
(74) Vertreter: Seemann & Partner Patentanwälte mbB
(86) Internationale Anmeldenummer: PCT/EP2017/070660
(87) Internationale Veröffentlichungsnummer: WO 2018/041626

(56) Entgegenhaltungen:
- WO-A1-2016/121171
- DE-A1-102012 020 934
- DE-A1-102012 218 754
- DE-U1-202004 010 580
- US-A1- 2006 252 991

## Beschreibung

Die Erfindung betrifft ein Verfahren zur Dichtigkeitsprüfung eines chirurgischen Instruments, insbesondere Endoskops, weiter vorzugsweise flexiblen Endoskops, in einer Aufbereitungsvorrichtung, wobei das chirurgische Instrument wenigstens einen zu reinigenden Kanal, insbesondere Arbeitskanal, aufweist und das chirurgische Instrument zur Dichtigkeitsprüfung mit einem Druckluftschlauch druckluftdicht verbunden wird, wobei vor einer Aufbereitung des chirurgischen Instruments mittels wenigstens einer Reinigungsflüssigkeit der Druckluftschlauch mittels Trocknungsluft aus einer Trocknungsvorrichtung der Aufbereitungsvorrichtung beaufschlagt wird, so dass anhand der eingesetzten Trocknungsluft die Dichtigkeitsprüfung des chirurgischen Instruments durchgeführt wird.

Zur Aufbereitung von, insbesondere flexiblen, Endoskopen werden üblicherweise Reinigungs- und Desinfektionsgeräte (RDG-E) eingesetzt, die auf einem chemisch-thermischen Aufbereitungsprozess basieren. Um die Endoskope durch die Reinigungsflüssigkeit nicht zu beschädigen, ist es wichtig, dass die Hülle des Endoskops vollständig intakt ist und somit Reinigungsflüssigkeit nicht in das Innere des Endoskops eindringen kann. Dies wird im RDG-E vor dem ersten Wassereinlauf überprüft, indem das Endoskop über eine entsprechende Schnittstelle auf einen Überdruck aufgepumpt wird. Anschließend wird der Druckabfall überwacht, wobei aus einem Druckabfall auf eine Leckage in dem Endoskop geschlossen werden kann. Bei fehlgeschlagener Prüfung bzw. zu Prozessende, also zum Ende des Reinigungs- und Desinfektionsprozesses, wird das Endoskop wieder teilweise entlüftet. Die Aufbereitung selbst erfolgt bei geringerem Überdruck im Endoskop.

Entsprechende RDG-E-Systeme der OLYMPUS Winter & Ibe GmbH, Hamburg, sind unter der Bezeichnung "ETD" bekannt. Diese Systeme umfassen einen sogenannten "Leak Tester", der den Dichtigkeitstest durchführt. Dazu wird ein Endoskop initial nach Programmstart zur Dichtigkeitsprüfung mit einem Überdruck von beispielsweise 285 mbar gegenüber dem Umgebungsdruck aufgepumpt und der Überdruck nach dem Dichtigkeitstest auf ca. 150 mbar über Umgebungsdruck reduziert. Dieser Druck wird während der Aufbereitung gehalten und kontinuierlich überwacht, auch um das Eindringen von Wasser von außen zu verhindern.

Bei der Verwendung der Endoskope in der Endoskopie treten vereinzelt Schäden auf, bei denen Endoskope von Biopsiekanälen her punktiert werden. Es entsteht eine Mikroperforation, die bei einem durchgeführten Dichtigkeitstest unter Umständen nicht erkannt wird. Bei der nachfolgenden Aufbereitung wird Spül- und Klarwasser mit einem deutlich größeren Druck als 150 mbar durch die zu spülenden Kanäle geleitet. Dadurch kann dann ein Übertrag von Wasser aus der Kanalspülung in den zu schützenden Bereich des Endoskops erfolgen, was zu einer Druckerhöhung im Endoskop über 150 mbar hinaus führt. Der Leak Tester reagiert darauf mit einem Ablassen von Druckluft aus dem Endoskop, um den Überdruck von 150 mbar zu halten. Dadurch kann Wasser über den Weg der Entlüftung in die Verschlauchung eindringen und schlussendlich in den Leak Tester eingetragen werden und diesen schlimmstenfalls zerstören.

Bei der Prüfung der Dichtigkeit von chirurgischen Instrumenten, z.B. Endoskopen, wird das Endoskop mit einer Dichtigkeitstesteinrichtung verbunden, um zu überprüfen, ob die äußere Hülle des Endoskops sowie die Kanäle des Endoskops vollständig intakt sind.

Vor dem ersten Reinigungsvorgang mit einer Flüssigkeit, wie z.B. Wasser, wird hierbei automatisch überprüft, indem die Dichtigkeitstesteinrichtung über einen entsprechenden Anschluss mit dem Endoskop verbunden wird und anschließend auf einen Überdruck aufgepumpt wird. Danach wird der Druckabfall überwacht, um eine Leckage im Endoskop festzustellen.

Aufgrund der Schnittstelle, die beispielsweise flexible Endoskope haben, um die Dichtigkeit der Endoskophülle zu überprüfen und die Endoskope zu entlüften, besteht die Gefahr, dass bei falscher Handhabung durch einen Anwender Wasser in die intakte Endoskophülle eindringt. Zudem können in den Endoskopkanälen Undichtigkeiten entstehen, die bei dem geringen Prüfdruck im Rahmen der Dichtigkeitsprüfung (ca. 300 mbar) nicht erkannt werden, bei den höheren Spüldrücken (ca. 1300 mbar) jedoch dafür sorgen, dass Wasser in die Endoskophülle eindringt, wodurch auch der Überdruck in der Endoskophülle (ca. 150 mbar) nicht verhindert werden kann. Dies kann dazu führen, dass das Endoskop beschädigt wird.

Sofern in einem undichten Endoskop Wasser vorhanden ist, kann dies dazu führen, dass das eingedrungene Wasser beim Entlüften in das Reinigungs- und Desinfektionsgerät (RDG-E) gelangen kann, wodurch dieses ebenfalls beschädigt wird.

Um die Undichtigkeit zu erkennen, wird vor der Durchführung des Reinigungsvorgangs des Endoskops das Endoskop auf einen Prüfdruck gebracht, typischerweise 275 mbar bis 300 mbar, und dann für eine bestimmte Zeit, z.B. 60 Sekunden, geprüft. Fällt der Druck innerhalb des Endoskops um mehr als einen zuvor festgelegten Betrag, typischerweise zwischen 10 mbar bis 15 mbar, ab, so wird das Endoskop als undicht angesehen, so dass die anschließende Aufbereitung nicht durchgeführt wird.

Darüber hinaus besteht bei den derzeitigen Dichtigkeitsprüfverfahren eine Ungenauigkeit darin, bestimmte Arten von Undichtigkeiten, vor allem bei richtungsabhängigen Undichtigkeiten, diese zuverlässig zu erkennen.

In US 2006/0252991 A1 sind ein System und ein Verfahren zur Bestimmung der Integrität eines Endoskops offenbart. Hier weist eine Testvorrichtung einen Kompressor auf, um Druckluft für die Kanäle in einem Endoskop bereitzustellen. Hierzu ist vorgesehen, dass in der Luftstrecke die Luft des Luftkompressors durch eine Filterreihe strömt, wobei die Filterreihe ein Trockengefäß umfasst. Vor der Aufbereitung des Endoskops wird hierbei getrocknete Luft aus dieser Trocknungsvorrichtung in die Arbeitskanäle geschickt.

Ferner offenbart DE 20 2004 010 580 U1 einen Sicherheitstester und einen Trockner für Endoskope, wobei ein Modul für die Trocknung vorgesehen ist. Hierbei ist vorgesehen, dass keine Luft mehr in das Endoskop gelangt, die nicht vorher durch die mit Trockenmitteln gefüllte Trockenmittelkammer geströmt ist. Somit wird bei einer Dichtigkeitsprüfung trockene Luft in die Arbeitskanäle gepumpt.

Außerdem ist in DE 10 2012 218 754 A1 ein Verfahren zum Betrieb einer Reinigungsmaschine von einem einen Kanal aufweisenden chirurgischen Instrument beschrieben. Hierbei ist ein Druckluftanschluss am Eingang des Kanals vorgesehen, wobei Druckluft zum Eingang des Kanals zugeführt wird. Hierbei wird ein Zuführdruckniveau der Druckluft durch Zuschalten oder Abschalten einer Druckluftversorgung verändert. Anschließend erfolgt das Messen des Drucks vor dem Eingang oder im Eingang des Kanals über eine vorgebbare Zeit und durch Bestimmung der Dichtigkeit des Kanals oder des Druckluftanschlusses an dem Kanal.

Zudem ist in WO 2016/121171 A1 (entspricht EP 3 111 823 A1) allgemein eine Vorrichtung zur Dichtigkeitsprüfung sowie eine Endoskopaufbereitungsvorrichtung gezeigt.

Darüber hinaus offenbart DE 10 2012 020 934 A1 eine Vorrichtung sowie ein Verfahren zum Reinigen und Desinfizieren von Endoskopen, wobei alternierend ein Fluid zu einer ersten Gruppe von Schläuchen zugeführt wird und die Zufuhr von einem Fluid zu einer zweiten Gruppe von Schläuchen abgesperrt wird. Für den Reinigungsprozess werden hierbei Ventile alternierend geöffnet und geschlossen. Nach Beendigung der Reinigungs- und Spülvorgänge werden die Kanäle mit Druckluft über eine Druckluftleitung durchgeblasen.

Ausgehend von diesem Stand der Technik besteht die Aufgabe der Erfindung darin, insbesondere bei der Durchführung eines automatischen Dichtigkeitstests in einer Aufbereitungseinrichtung, die Dichtigkeitsprüfung zu verbessern.

Gelöst wird diese Aufgabe durch ein Verfahren zur Dichtigkeitsprüfung eines chirurgischen Instruments, insbesondere Endoskops, weiter vorzugsweise flexiblen Endoskops, in einer Aufbereitungsvorrichtung, wobei das chirurgische Instrument wenigstens einen zu reinigenden oder zu spülenden Kanal, insbesondere Arbeitskanal, aufweist und das chirurgische Instrument zur Dichtigkeitsprüfung mit einem Druckluftschlauch druckluftdicht verbunden wird, wobei vor einer Aufbereitung des chirurgischen Instruments mittels wenigstens einer Reinigungsflüssigkeit der Druckluftschlauch mittels Trocknungsluft aus einer Trocknungsvorrichtung der Aufbereitungsvorrichtung beaufschlagt wird, so dass anhand der eingesetzten Trocknungsluft die Dichtigkeitsprüfung des chirurgischen Instruments durchgeführt wird, das dadurch weitergebildet wird, dass das chirurgische Instrument zusätzlich zum wenigstens einen Kanal, insbesondere Arbeitskanal, einen gegenüber dem Kanal geschützten Bereich, insbesondere Schaftbereich des chirurgischen Instruments, aufweist, wobei nach der Beaufschlagung des Kanals des chirurgischen Instruments mit Trocknungsluft bei Überschreiten eines vorbestimmten Drucksollwerts für den geschützten Bereich eine Undichtigkeit des chirurgischen Instruments festgestellt wird.

Die Erfindung beruht auf dem Gedanken, dass eine Aufbereitungsvorrichtung zum Reinigen und/oder Desinfizieren von chirurgischen Instrumenten bzw. Endoskopen mit einer Trocknungsvorrichtung zum Trocknen der Kanäle, insbesondere Arbeitskanäle, des chirurgischen Instruments bzw. Endoskops ausgebildet ist, wobei die Trocknungsvorrichtung für die initiierte Dichtigkeitsprüfung des chirurgischen Instruments bzw. Endoskops eingesetzt bzw. verwendet wird, so dass die automatische Dichtigkeitsprüfung unter Verwendung der Trocknungsluft der Trocknungsvorrichtung in der Aufbereitungsvorrichtung ausgeführt wird.

Die Trocknungsvorrichtung stellt die Trocknungsluft bereit. Trocknungsluft ist getrocknet und im Vergleich zu herkömmlicher Druckluft trockner, weist also eine geringere Feuchtigkeit auf. Es kann vorgesehen sein, dass die Trocknungsvorrichtung warme oder erwärmte Trocknungsluft bereitstellt. Die Trocknungsvorrichtung weist beispielsweise einen Filter oder dergleichen auf, in welchem die der Luft entzogene Feuchtigkeit absorbiert wird. Die Trocknungsvorrichtung ist von einer Druckluftquelle zu unterscheiden. Eine Druckluftquelle dient der Bereitstellung von Druckluft, also Luft, welche keinen Trocknungsvorgang durchlaufen hat.

Hierbei wird bei der Durchführung des Dichtigkeitstests der zu reinigende oder zu spülende Kanal, insbesondere Arbeitskanal, mit Trocknungsluft beaufschlagt, wobei die Trocknungsluft durch den Kanal geführt wird. Bei Auftreten einer Undichtigkeit im Arbeitskanal bzw. Innenkanal würde dies zu einem Druckanstieg in einem geschützten Bereich des chirurgischen Instruments führen, so dass anhand des erfassten Druckanstiegs im gegenüber dem Kanal geschützten Bereich festgestellt wird, dass das chirurgische Instrument undicht ist.

Insbesondere handelt es sich bei der Aufbereitungsvorrichtung mit der Trocknungsvorrichtung beispielsweise um ein Reinigungs- und Desinfektionsgerät (RDG-E).

Dazu ist in einer Weiterbildung vorgesehen, dass das chirurgische Instrument mittels eines Druckluftimpulses mit Trocknungsluft mit einer Dauer von weniger als 20 Sekunden, insbesondere zwischen 1 Sekunde bis 15 Sekunden, beaufschlagt wird. Für die Prüfung der Dichtigkeit mittels des Druckluftimpulses ist es ausreichend, dass kurzfristig die Trocknungsvorrichtung aktiviert wird, um eine eventuelle Undichtigkeit des chirurgischen Instruments zu ermitteln.

Des Weiteren ist es bei einer bevorzugten Ausführungsform des Verfahrens vorteilhaft, dass das chirurgische Instrument mit Trocknungsluft mit einem Druck von über 500 mbar, vorzugsweise über 800 mbar, weiter vorzugsweise über 1.000 mbar, beaufschlagt wird. Hierzu ist vorgesehen, dass das chirurgische Instrument mit einem Druck von über 500 mbar oder 600 mbar oder 700 mbar oder 800 mbar oder 900 mbar oder 1.000 mbar oder 1.100 mbar oder 1.200 mbar beaufschlagt wird.

Gemäß der Erfindung ist vorgesehen, dass das chirurgische Instrument zusätzlich zum wenigstens einen Kanal, insbesondere Arbeitskanal, einen gegenüber dem Kanal geschützten Bereich, insbesondere Schaftbereich des chirurgischen Instruments, weiter insbesondere Endoskopschaftbereich, aufweist, wobei nach der Beaufschlagung des Kanals des chirurgischen Instruments mit Trocknungsluft bei Überschreiten eines vorbestimmten Drucksollwerts für den geschützten Bereich eine Undichtigkeit des chirurgischen Instruments festgestellt wird. Dadurch, dass für die Dichtigkeitsprüfung der eingesetzte Druck der Trocknungsluft höher ist als der bei der Reinigung verwendete Wasserdruck, wird bei Überschreiten eines vorbestimmten Drucksollwerts für den gegenüber dem Kanal geschützten Bereich des chirurgischen Instruments die Undichtigkeit des chirurgischen Instruments ermittelt.

Insgesamt wird dadurch die Sicherheit bei der Erkennung von Undichtigkeiten deutlich erhöht, da die Undichtigkeiten vor Beginn der Aufbereitung des chirurgischen Instruments in der Aufbereitungsvorrichtung sicher erkannt werden. Da bei dem erfindungsgemäßen Verfahren die Druckdifferenz höher ist als bei dem bislang im Stand der Technik bekannten Verfahren, wird die Betriebssicherheit der Aufbereitungsvorrichtung signifikant erhöht.

Dazu ist in einer Ausgestaltung weiter vorgesehen, dass nach Feststellen der Undichtigkeit des chirurgischen Instruments der weitere Aufbereitungsprozess für das chirurgische Instrument gestoppt wird. Vorzugsweise wird durch Unterschreiten eines oder des vorbestimmten Drucksollwerts für den geschützten Bereich des chirurgischen Instruments die Aufbereitung des chirurgischen Instruments fortgesetzt.

Weitere Merkmale der Erfindung werden aus der Beschreibung erfindungsgemäßer Ausführungsformen zusammen mit den Ansprüchen und der beigefügten Zeichnung ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand eines Ausführungsbeispiels unter Bezugnahme auf die Zeichnung beschrieben, wobei bezüglich aller im Text nicht näher erläuterten erfindungsgemäßen Einzelheiten ausdrücklich auf die Zeichnung verwiesen wird. Es zeigt:

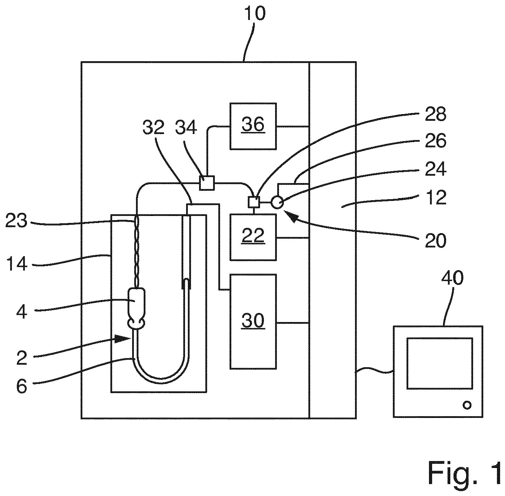

- Fig. 1: eine schematische Darstellung eines Reinigungsund Desinfektionsgeräts.

Fig. 1 zeigt eine schematische Darstellung eines Aufbereitungssystems 10 mit einer Reinigungskammer 14, in der ein flexibles Endoskop 2 angeordnet ist, das bereit ist, gereinigt und desinfiziert zu werden. Das Endoskop 2 umfasst einen Griff 4 sowie einen flexiblen Schaft 6, die beide an Anschlüsse des Aufbereitungssystems 10 angeschlossen sind.

Der Schaft 6 des Endoskops 2 ist über einen Anschluss mit einer Aufbereitungsvorrichtung 30 des Aufbereitungssystems 10 verbunden, während der Handgriff 4 über einen Druckluftschlauch 23 mit einer Druckluftquelle 22, beispielsweise einem Kompressor, mit der Vorrichtung 20 zur Druckluftbeaufschlagung, kurz "Leakage Tester" genannt, verbunden ist. Diese ist, ebenso wie die Aufbereitungsvorrichtung 30, mit einer Steuervorrichtung 12 des Aufbereitungssystems 10 verbunden, die somit auch eine Steuervorrichtung für die Vorrichtung 20 zur Druckluftbeaufschlagung darstellt. Die Druckluftquelle 22 dient dem Bereitstellen von Druckluft.

Ausgangs der Druckluftquelle 22 ist ein Luftdrucksensor 24 an den Druckluftschlauch 23 angeschlossen, der den Überdruck der Luft in dem Druckluftschlauch 23 über dem Umgebungsdruck misst und entsprechende Signale über eine Signalleitung 26 ebenfalls an die Steuervorrichtung 12 des Aufbereitungssystems 10 übermittelt. Die Steuervorrichtung 12 ist außerdem mit einer Anzeigevorrichtung 40 verbunden, mittels der die Steuervorrichtung 12 von außen beeinflusst werden kann und umgekehrt Daten aus der Steuervorrichtung 12, beispielsweise Überdruckmessdaten von dem Luftdrucksensor 24 und/oder Prozessdaten, angezeigt werden können.

Die Vorrichtung 20 zur Druckluftbeaufschlagung ist mit einer Druckluftquelle 22 über einen Druckluftschlauch 23 mit einem nur schematisch angedeuteten Endoskop 2 verbunden, wodurch Druckluft in das Endoskop 2 über ein Ventil 28 und ein in Durchlassstellung geschaltetes Ventil 34 gepumpt wird und danach auch wieder ausgelassen wird.

Mit dem Druckluftschlauch 23 ist ein Luftdrucksensor 24 verbunden, der den Druck der Druckluft im Druckluftschlauch 23 und im Endoskop 2 misst. Der Luftdrucksensor 24 kann alternativ auch am Endoskop 2 direkt messen oder am Ausgang der Druckluftquelle 22.

Ferner weist das Aufbereitungssystem 10 eine Trocknungsvorrichtung 36 auf, die dafür vorgesehen und eingerichtet ist, nach den Spül- und/oder Desinfektionsprozessen, die nach einer bestandenen Dichtigkeitsprüfung des Endoskops 2 durchgeführt werden, den oder die Kanäle des Endoskops 2 mit Trocknungsluft zu beaufschlagen und den oder die Kanäle des Endoskops 2 zu trocknen. Die Trocknungsvorrichtung 36 dient zum Bereitstellen von Trocknungsluft. Trocknungsluft weist eine verringerte Feuchtigkeit auf, hierzu ist die Trocknungsvorrichtung 36 beispielsweise mit einem Filter ausgestattet, in welchem Feuchtigkeit absorbiert wird. Im Vergleich zu herkömmlicher Druckluft, wie sie beispielsweise von der Druckluftquelle 22 bereitgestellt wird, handelt es sich bei der von der Trocknungsvorrichtung 36 bereitgestellten Trocknungsluft um Luft mit einer geringeren Feuchtigkeit.

Darüber hinaus wird die Trocknungsvorrichtung 36 vor der Durchführung der Spül- und/oder Desinfektionsprozesse dafür verwendet, um bei der Durchführung der initialen Dichtigkeitsprüfung das Endoskop bzw. den Kanal oder die Kanäle des Endoskops 2 mit Trocknungsluft zu beaufschlagen. Hierbei wird die Trocknungsluft aus der Trocknungsvorrichtung 36 über das Ventil 34 in den Druckluftschlauch 23 geleitet. Hierbei wird die Trocknungsluft beispielsweise mit einem Druck über 800 mbar oder 1.000 mbar oder mehr beaufschlagt. Insbesondere wird hierbei die Trocknungsluft mittels eines Impulses, vorzugsweise mit einer Impulsdauer von einer Sekunde bis 15 Sekunden, in das Endoskop 2 bzw. dessen Kanal oder Kanäle geleitet. Anschließend wird das Ventil 28 derart gestaltet, dass die Trocknungsluft über den Druckluftschlauch 23 in den Luftdrucksensor 24 für die Dichtigkeitsprüfung geleitet wird.

Hierbei wird vorzugsweise der Druck bzw. Luftdruck in einem gegenüber einem Spülkanal durch eine Hülle geschützten Bereich erfasst. Wird mittels des Luftdrucksensors 24 festgestellt, dass nach der Beaufschlagung des Endoskops 2 mit Druckluft einen vorbestimmten Sollwert überschreitet, wird mittels der Steuereinheit 12 festgestellt, dass eine Undichtigkeit des Endoskops 2 vorliegt. In diesem Fall wird der Aufbereitungsprozess in dem Aufbereitungssystem 10 gestoppt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Im Rahmen der Erfindung sind Merkmale, die mit "insbesondere" oder "vorzugsweise" gekennzeichnet sind, als fakultative Merkmale zu verstehen.

### Bezuqszeichenliste

- 2: Endoskop
- 4: Griff
- 6: flexibler Schaft
- 10: Aufbereitungssystem
- 12: Steuereinheit
- 14: Reinigungskammer
- 20: Vorrichtung zur Druckluftbeaufschlagung
- 22: Druckluftquelle
- 23: Druckluftschlauch
- 24: Luftdrucksensor
- 26: Signalleitung
- 28: Ventil
- 30: Aufbereitungsvorrichtung
- 32: Spülschlauch
- 34: Ventil
- 36: Trocknungsvorrichtung
- 40: Anzeigeeinheit

## Patentansprüche

1. Verfahren zur Dichtigkeitsprüfung eines chirurgischen Instruments, insbesondere Endoskops, weiter vorzugsweise flexiblen Endoskops, (2) in einer Aufbereitungsvorrichtung (10), wobei das chirurgische Instrument (2) wenigstens einen zu reinigenden Kanal, insbesondere Arbeitskanal, aufweist und das chirurgische Instrument (2) zur Dichtigkeitsprüfung mit einem Druckluftschlauch (23) druckluftdicht verbunden wird, wobei vor einer Aufbereitung des chirurgischen Instruments (2) mittels wenigstens einer Reinigungsflüssigkeit der Druckluftschlauch (23) mittels Trocknungsluft aus einer Trocknungsvorrichtung (36) der Aufbereitungsvorrichtung (10) beaufschlagt wird, so dass anhand der eingesetzten Trocknungsluft die Dichtigkeitsprüfung des chirurgischen Instruments (2) durchgeführt wird, wobei das chirurgische Instrument (2) zusätzlich zum wenigstens einen Kanal, insbesondere Arbeitskanal, einen gegenüber dem Kanal geschützten Bereich, insbesondere Schaftbereich des chirurgischen Instruments, aufweist, **dadurch gekennzeichnet, dass** nach der Beaufschlagung des Kanals des chirurgischen Instruments (2) mit Trocknungsluft bei Überschreiten eines vorbestimmten Drucksollwerts für den geschützten Bereich eine Undichtigkeit des chirurgischen Instruments (2) festgestellt wird.

2. Verfahren nach Anspruch 1, **dadurch gekennzeichnet, dass** das chirurgische Instrument (2) mittels eines Trocknungsluftimpulses mit Trocknungsluft mit einer Dauer von weniger als 20 Sekunden, insbesondere zwischen 1 Sekunde bis 15 Sekunden, beaufschlagt wird.

3. Verfahren nach Anspruch 1 oder 2, **dadurch gekennzeichnet, dass** das chirurgische Instrument (2) mit Trocknungsluft mit einem Druck von über 500 mbar, vorzugsweise über 800 mbar, weiter vorzugsweise über 1.000 mbar, beaufschlagt wird.

4. Verfahren nach einem der Ansprüche 1 bis 3, **dadurch gekennzeichnet, dass** nach Feststellen der Undichtigkeit des chirurgischen Instruments (2) der weitere Aufbereitungsprozess für das chirurgische Instrument (2) gestoppt wird.

5. Verfahren nach einem der Ansprüche 1 bis 3, **dadurch gekennzeichnet, dass** bei Unterschreiten eines oder des vorbestimmten Drucksollwerts für den geschützten Bereich die Aufbereitung des chirurgischen Instruments (2) fortgesetzt wird.

## Claims

1. A method for leak testing a surgical instrument, in particular an endoscope, further preferably a flexible endoscope (2), in a reprocessing apparatus (10), wherein the surgical instrument (2) has at least one channel to be cleaned, in particular a working channel, and the surgical instrument (2) is connected to a compressed air hose (23) tight to compressed air for the leak test, wherein drying air is applied to the compressed air hose (23) from a drying apparatus (36) of the reprocessing apparatus (10) prior to a reprocessing the surgical instrument (2) using at least one cleaning liquid such that the leak test of the surgical instrument (2) is performed by means of the utilized drying air, wherein the surgical instrument (2) has a region that is protected from the channel, in particular a shaft region of the surgical instrument, in addition to the at least one channel, in particular working channel, **characterized in that** after drying air is applied to the channel of the surgical instrument (2), a leak in the surgical instrument (2) is determined when a predetermined pressure setpoint of the protected region is exceeded.

2. The method according to claim 1, **characterized in that** drying air is applied to the surgical instrument (2) by means of a pulse of drying air with a duration of less than 20 seconds, in particular between 1 second to 15 seconds.

3. The method according to claim 1 or 2, **characterized in that** drying air is applied to the surgical instrument (2) at a pressure greater than 500 mbar, preferably greater than 800 mbar, further preferably greater than 1000 mbar.

4. The method according to any of the claims 1 to 3, **characterized in that** after the leak in the surgical instrument (2) is determined, the additional reprocessing process for the surgical instrument (2) is stopped.

5. The method according to any of the claims 1 to 3, **characterized in that** the reprocessing of the surgical instrument (2) is continued when a or the predetermined pressure setpoint for the protected region is undershot.

## Revendications

1. Procédé de contrôle d'étanchéité d'un instrument chirurgical, en particulier d'un endoscope, de façon encore préférée d'un endoscope flexible (2) dans un dispositif de retraitement (10), dans lequel l'instrument chirurgical (2) présente au moins un conduit à nettoyer, en particulier un conduit de travail, et l'instrument chirurgical (2) étant relié de manière étanche à l'air comprimé à un tuyau d'air comprimé (23) pour le contrôle d'étanchéité ; avant le retraitement de l'instrument chirurgical (2) au moyen d'au moins un liquide de nettoyage, le tuyau d'air comprimé (23) est alimenté avec de l'air de séchage provenant d'un dispositif de séchage (36) du dispositif de retraitement (10), afin que le contrôle d'étanchéité de l'instrument chirurgical (2) soit effectué au moyen de l'air de séchage utilisé, l'instrument chirurgical (2) présentant, en plus dudit au moins un conduit, en particulier du conduit de travail, une zone protégée par rapport au conduit, en particulier une zone de tige de l'instrument chirurgical, **caractérisé en ce qu'**après que le conduit de l'instrument chirurgical (2) ait été exposé à l'air de séchage, une fuite de l'instrument chirurgical (2) est détectée lors du dépassement d'une valeur de consigne de pression prédéterminée pour la zone protégée.

2. Procédé selon la revendication 1, **caractérisé en ce que** l'instrument chirurgical (2) est exposé à de l'air de séchage, au moyen d'une impulsion d'air de séchage, pendant une durée inférieure à 20 secondes, en particulier comprise entre 1 seconde et 15 secondes.

3. Procédé selon la revendication 1 ou la revendication 2, **caractérisé en ce que** l'instrument chirurgical (2) est exposé à de l'air de séchage sous une pression supérieure à 500 mbar, de préférence supérieure à 800 mbar, de façon encore préférée, supérieure à 1000 mbar.

4. Procédé selon l'une des revendications 1 à 3, **caractérisé en ce qu'**après la détection de la fuite de l'instrument chirurgical (2), la suite du processus de retraitement de l'instrument chirurgical (2) est arrêtée.

5. Procédé selon l'une des revendications 1 à 3, **caractérisé en ce que** le retraitement de l'instrument chirurgical (2) est poursuivi lorsque la pression descend en dessous d'une ou des consignes de pression prédéterminées pour la zone protégée.
